# EUROPÄISCHE PATENTSCHRIFT

(11) **EP 2 061 987 B1**
(45) Veröffentlichungstag und Bekanntmachung des Hinweises auf die Patenterteilung: **31.10.2012**
(21) Anmeldenummer: 07818143.5
(22) Anmeldetag: 13.09.2007
(51) Int. Cl.: F17B 1/26, C12M 1/107

(54) **GASSPEICHER SOWIE BIOGASANLAGE MIT EINEM GASSPEICHER**
GAS RESERVOIR AND BIOGAS INSTALLATION COMPRISING A GAS RESERVOIR
RESERVOIR DE GAZ ET INSTALLATION DE BIOGAZ MUNIE D'UN RESERVOIR DE GAZ

(30) Priorität: 15.09.2006 DE 202006014148 U
(43) Veröffentlichungstag der Anmeldung: 27.05.2009
(73) Patentinhaber: Reinelt, Gerhard, 49124 Georgsmarienhütte (DE)
(72) Erfinder: Reinelt, Gerhard, 49124 Georgsmarienhütte (DE)
(74) Vertreter: Pott, Ulrich
(86) Internationale Anmeldenummer: PCT/EP2007/007980
(87) Internationale Veröffentlichungsnummer: WO 2008/031593

(56) Entgegenhaltungen:
- DE-A1- 3 735 208
- DE-U1- 20 208 178
- FR-A- 680 304

## Beschreibung

Die Erfindung betrifft einen Gasspeicher nach dem Oberbegriff des Anspruchs 1 sowie eine Biogasanlage zur Erzeugung von Biogas, insbesondere Methan, mit einem derartigen Gasspeicher.

Aus der DE 202 08 171 U ist ein konstruktiv einfacher und relativ zum Gebrauchswert preisgünstiger Gasbehälter bekannt. Dieser weist einen zylindrischen Gasraum auf, der seitlich von einer flexiblen Wandung, unterseitig von einem Boden und oberseitig von einer Decke begrenzt wird. Beim Befüllen des Gasraumes mit Gas wird die Decke entgegen deren Gewichtskraft durch den Gasdruck angehoben und entfernt sich zunehmend vom Boden. Der Gasraum wird dadurch größer. Der Wandung sind mehrere Ausdehnungsbegrenzer zugeordnet, die mit der Wandung beweglich sind und deren Formgebung zwangsbeeinflussen. Die Ausdehnungsbegrenzer stabilisieren die Wandung des Gasspeichers.

Der beschriebene Gasbehälter arbeitet im Prinzip sehr zuverlässig. Im befüllten Zustand des Gasbehälters kann es jedoch infolge seitlich angreifender Winde oder infolge manuellen Eingreifens dazu kommen, daß die Wandungen des Gasbehälters zusammen mit den Ausdehnungsbegrenzern horizontal zur Seite hin ausweichen und der Betrieb dadurch gestört wird.

Um ein derartiges Ausweichen der Wandung zur Seite zu unterbinden, ist die Anordnung eines Käfigs um den Gasbehälter herum im Stand der Technik vorgeschlagen worden. Ein derartiger Gasbehälter ist aus der FR 680304A , dass die Merkmale des Oberbegriffs des Anspruchs 1 zeigt, bereits bekannt.

Diese Konstruktion hat jedoch den Nachteil, daß die Wandung des Gasbehälters, sofern diese seitlich ausweicht und die Ausweichbewegung vom Käfig gestoppt wird, an dem Käfig entlang streifen und infolge von Reibungskräften mit der Zeit abnutzen kann.

Der Erfindung liegt die Aufgabe zugrunde, einen Gasspeicher der eingangs genannten Art zu entwickeln, der konstruktiv einfach aufgebaut ist und auch im dauerhaften Betrieb zuverlässig arbeitet sowie eine Biogasanlage mit einem derartigen Gasspeicher bereit zu stellen.

Die Erfindung löst diese Aufgabe durch einen Gasspeicher mit den Merkmalen des Anspruchs 1 sowie durch eine Biogasanlage mit den Merkmalen des Anspruchs 15. Vorteilhafte Ausgestaltungen ergeben sich aus den abhängigen Ansprüchen 2 bis 14 und 16.

Das Stabilisierungselement ist flexibel ausgebildet und je nach Füllzustand des Gasraumes ganz oder teilweise mit Hilfe einer Spanneinrichtung zwischen einem oder mehreren Paaren zusammenwirkender Widerlager durch Zugspannung gestrafft. Der gestraffte Teil des Stabilisierungselements verhindert ein seitliches Ausbrechen der Wandung. Da zusätzlich lediglich eines der jeweils zusammenwirkenden Widerlager fest mit einem der beweglichen Teile verbunden ist, wird der jeweils gestraffte Teil des Stabilisierungselements auch bei einer Änderung der Größe des Gasraumes in seiner relativen Position zur Wandung des Gasraumes nicht verändert. Hierdurch wird sichergestellt, daß die Wandung nicht an dem Stabilisierungselement abgerieben wird. Ein dauerhaft zuverlässiger Betrieb des Gasspeichers ist so sichergestellt.

Das Stabilisierungselement wird bevorzugt von einem Netz, einem Seil, einem Gewebe, einer Folie oder dergleichen gebildet. Es sind jedoch auch Kombinationen zweier oder mehrerer dieser Ausgestaltungen des Stabilisierungselementes möglich.

Die Spanneinrichtung beaufschlagt das Stabilisierungselement mit Vorteil mit einer Zugspannung, die im wesentlichen parallel zur Wandung des Gasspeichers ausgerichtet ist. Die Zugspannung bildet dabei eine Gegenkraft zur Ausdehnung der Wandung. Im Falle einer zylindrischen Form des Gasraumes mit einer im wesentlichen vertikal ausgerichteten Wandung ist auch das Stabilisierungselement bevorzugt in vertikaler Richtung mit einer Zugspannung beaufschlagt. Die Zugspannung wird bevorzugt von einem direkt oder indirekt am Stabilisierungselement angreifenden Zuggewicht und/oder wenigstens einer Feder auf das Stabilisierungselement übertragen. Das Zuggewicht kann dabei von einem separaten Element oder aber kostensparend von der Decke des Gasspeichers gebildet werden.

An der Decke sind mit Vorteil Halterungen zur Anbringung zusätzlicher Gewichte an oder auf der Decke vorgesehen. Hierdurch kann der Betriebsdruck im Inneren des Gasraumes erhöht werden, ohne die auf die Stabilisierungselemente einwirkende Zugspannung erhöhen zu müssen. Umgekehrt kann jedoch auch die Zugspannung erhöht werden ohne den Betriebsdruck im Inneren des Gasraumes erhöhen zu müssen, indem die Zuggewichte erhöht und gleichzeitig die an den Halterungen befestigten Gewichte der Decke reduziert werden. Eine gezielte und variable Regulierung des Betriebsdruckes und der Zugspannung der Stabilisierungselemente ist dadurch in einfacher Weise gewährleistet.

Die Decke kann von einer Platte gebildet werden. Um Gewicht und Kosten zu sparen, kann sie jedoch auch aus einem Gestell, beispielsweise einem Tragring, bestehen, das zum Gasraum hin mit einer gasdichten Membran unterlegt ist, wobei diese Membran gasdicht mit der Wandung verbunden ist. Hierdurch ist der Transport und Aufbau des Gasspeichers deutlich vereinfacht.

In einer bevorzugten Ausgestaltung der Erfindung weist die Wandung wenigstens eine Falthilfe auf, mit deren Hilfe eine gleichmäßige Faltung der Wandung bei einem sich verkleinernden Gasraum ermöglicht wird. Die Falthilfe kann dabei entweder mit der Wandung verbunden sein, beispielsweise in Form um die Wandung und parallel zum Boden laufender Ringe. Die Falthilfe kann jedoch auch Teil der Wandung in Form von Materialverstärkungen und Materialschwächungen an ausgewählten Stellen sein.

Weitere Ausgestaltungen und Vorteile der Erfindung ergeben sich aus den Unteransprüchen und den nachfolgend beschriebenen Ausführungsbeispielen in den Figuren; es zeigen:
- Fig. 1: einen erfindungsgemäßen Gasspeicher mit zwei Stabilisierungselementen in teilweise geschnittener Seitenansicht,
- Fig. 2: den Gegenstand aus Fig. 1 mit fast leerem Gasraum,
- Fig. 3: einen erfindungsgemäßen Gasspeicher mit acht Stabilisierungselementen in der Draufsicht,
- Fig. 4: eine erfindungsgemäße Biogasanlage mit gefülltem Gasspeicher in teilweise geschnittener Seitenansicht,
- Fig. 5: den Gegenstand aus Fig. 4 mit fast leerem Gasspeicher und
- Fig. 6: eine Vergrößerung des Ausschnittes A aus Fig. 5.

Nachfolgend werden gleichwirkende Elemente mit einem einheitlichen Bezugszeichen versehen.

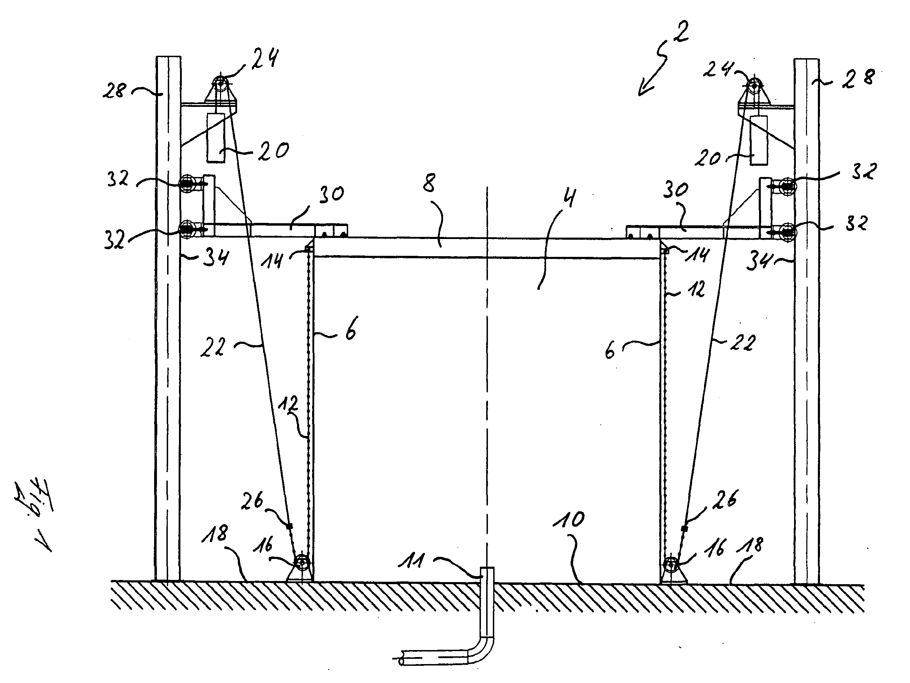

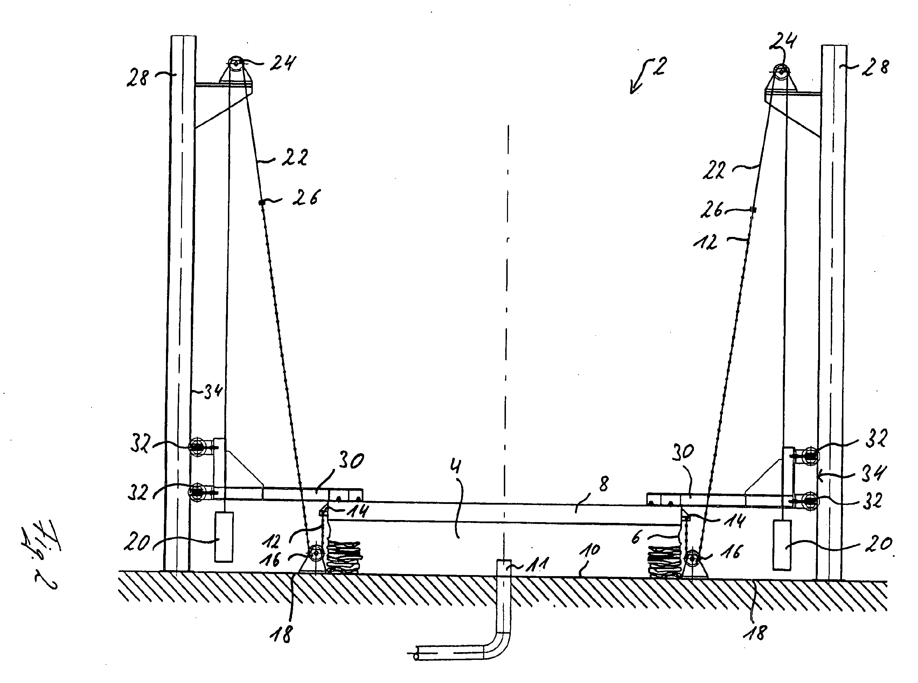

Die Figuren 1 und 2 zeigen einen erfindungsgemäßen Gasspeicher 2 mit einem größenvariablen Gasraum 4, der in Fig. 1 gefüllt und in Fig. 2 fast entleert ist. Der Gasraum 4 wird seitlich durch eine flexible Wandung 6 mit einer zylindrischen Grundform und oberseitig durch eine mit der Wandung 6 gasdicht verbundene Decke 8 begrenzt. Unterseitig wird der Gasraum 4 durch einen Boden 10 begrenzt. Die Befüllung des Gasraumes 4 mit Gas und die Entnahme von Gas erfolgt über einen im Boden 10 eingelassenen Gasanschluß 11.

Die Wandung 6 und die Decke 8 sind als bewegliche Teile ausgebildet, wie aus dem Vergleich der Figuren 1 und 2 ersichtlich ist. Die Ausdehnung der Wandung 6 wird seitlich durch Stabilisierungselemente 12 begrenzt. Die Stabilisierungselemente 12 sind gleichmäßig um die Wandung 6 herum verteilt. In Fig. 1 und 2 sind jeweils die linke und die rechte abgebildete Seite des Gasspeichers 2 gleich aufgebaut: Nachfolgend wird der Einfachheit halber gelegentlich lediglich eine der Seiten erläutert. Die Erläuterung gilt jedoch gleichermaßen auch für die andere Seite des Gasspeichers 2.

Das Stabilisierungselement 12 jeder Seite ist in sich flexibel und wird in dem Ausführungsbeispiel von einem Netz gebildet. Der Gasspeicher 2 weist in dem in Fig. 1 und 2 dargestellten Ausführungsbeispiel auf jeder Seite des Gasspeichers eine Spanneinrichtung mit jeweils einem Paar zusammenwirkender Widerlager 14, 16 auf. Ein Teil des Stabilisierungselements 12 ist zwischen den zusammenwirkenden Widerlagern 14, 16 durch eine Zugspannung gespannt. Die jeweils oberen Widerlager 14 sind fest mit der Decke als einem beweglichen Teil verbunden, wohingegen die unteren Widerlager nicht an einem der beweglichen Teile sondern am Boden 18 außerhalb des Gasraumes 4 seitlich neben der Wandung 6 befestigt sind. Die unteren Widerlager 16 sind als Umlenkrollen 16 ausgebildet.

Die Spanneinrichtung jeder Seite beaufschlagt das Stabilisierungselement 12 mit einer Zugspannung, die im wesentlichen parallel zur Wandung 6 des Gasspeichers 2 in dessen mit Gas befülltem Zustand ausgerichtet ist. Die Wandung 6 wird durch die unter Zugspannung stehenden Netze 12 im Bereich zwischen den Widerlagern 14, 16 an einer Ausdehnung oder Verlagerung über die Netze 12 hinaus gehindert. Gleichzeitig wird durch die Anordnung sichergestellt, daß die an die Stabilisierungselemente 12 angelehnte Wandung 6 bei einer Größenänderung des Gasraumes 4 nicht an den Stabilisierungselementen 12 entlang reibt und die Wandung 6 dadurch beschädigt wird. Die relative Position der Wandung 6 zu den gestrafften Teilen der Stabilisierungselemente 12 neben der Wandung 6 bleibt bei einer Änderung der Größe des Gasraumes 4 stets gleich. Wandung 6 und das diese an einer Dehnung nach außen begrenzende gestraffte Stabilisierungselement 12 führen während einer Größenänderung des Gasraumes 4 die gleiche Bewegung relativ zum Boden 10 oder 18 aus.

Die Spanneinrichtung jeder Seite weist jeweils ein Zuggewicht 20 auf, das zur Aufbringung der Zugkraft indirekt über ein Zugelement 22 an dem Stabilisierungselement 12 angreift, wobei das Stabilisierungselement 12 um die Umlenkrolle 16 geführt wird. Das Zuggewicht 20 kann jedoch auch direkt an dem Stabilisierungselement 12 angreifen. Auch kann das Zuggewicht von der Decke selbst gebildet werden, wodurch das Gesamtgewicht und die benötigte Materialmenge des Gasspeichers kostenreduzierend weiter gesenkt werden kann. Beide vorgenannten Ausgestaltungen sind in den Figuren jedoch nicht dargestellt.

Den Umlenkrollen 16 sind jeweils weitere Umlenkrollen 24 zugeordnet, die weiter vom Boden 18 entfernt angeordnet sind als die jeweils erste Umlenkrolle 16. Das Stabilisierungselement 12 ist ausgehend von dessen Befestigung an der Decke 8 als weiterem Widerlager 14 um die jeweils erste Umlenkrolle 16 geführt. Die Zugelemente 22 sind jeweils über Verbindungsmittel 26 mit dem Stabilisierungselement 12 verbunden und werden über die weitere Umlenkrolle 24 bis zu dem endseitigen Zuggewicht 20 geführt, das mit dem Zugelement 22 verbunden ist.

Im Ergebnis wird die Decke 8 einerseits von dem im Gasraum 4 herrschenden Betriebsdruck von etwa 50 Millibar über dem Außendruck vom Boden 10 weggedrückt. Diesem Druck wirkt andererseits das Eigengewicht der Decke 8 entgegen. Zudem wird die Decke 8 über die Stabilisierungselemente 12 sowie die daran angreifenden Zugelemente 22 durch die Zuggewichte 20 in Richtung Boden 10 des Gasraumes 4 gezogen. Die Decke 8 arbeitet bezüglich des Gasraumes 4 ähnlich einem Kolben.

Die weitere Umlenkrolle 24 ist an einer Stütze 28 befestigt, die neben dem Gasraum 4 steht. An der Decke 8 sind zwei Streben 30 mit endseitigen Führungsmitteln 32 in Form von jeweils zwei Rollen angeordnet. Die Führungsmittel 32 sind entlang diesen zugeordneter und komplementär zu diesen ausgebildeter Führungsschienen 34 verfahrbar. Die Führungsschienen 34 sind im Ausführungsbeispiel an den Stützen 28 angeordnet. Hierdurch kann Material und damit Kosten eingespart werden. Im Ausführungsbeispiel sind je Strebe 30 zwei Rollen als Führungsmittel 32 vorgesehen. Die Streben 30 bzw. die daran befestigten endseitigen Rollen 32 sind dabei so zur Decke 8 ausgerichtet, daß eine ungewollte Schrägstellung der Decke 8, zum Beispiel in Folge nicht gleichmäßig laufender Umlenkrollen 16, 24, relativ zur Wandung 6 verhindert wird.

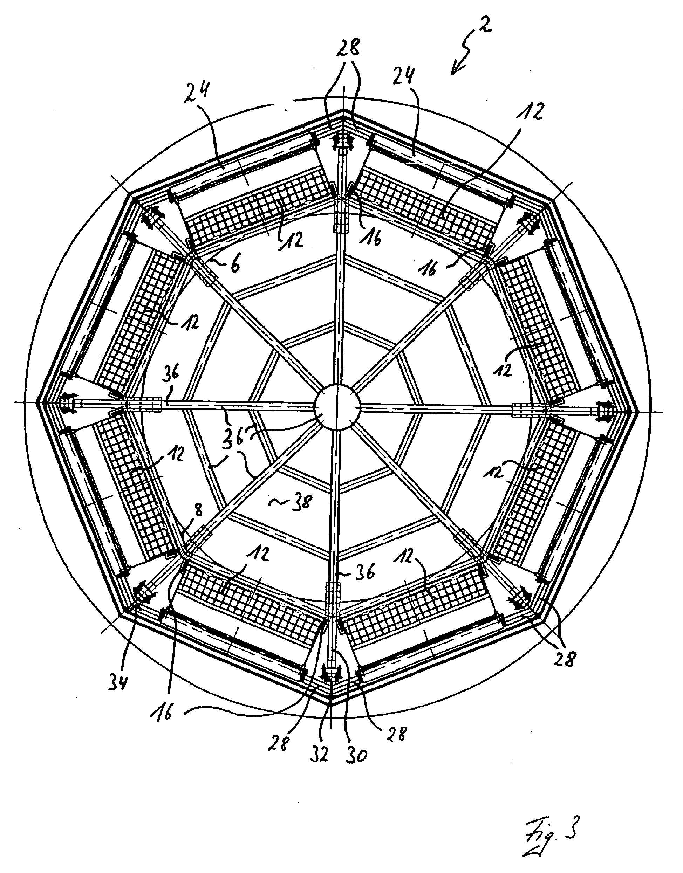

Fig. 3 zeigt eine weitere Ausgestaltung eines erfindungsgemäßen Gasspeichers 2 in der Draufsicht. Der Gasspeicher 2 weist acht Stabilisierungselemente 12 und entsprechend acht erste Umlenkrollen 16 auf. Die Stabilisierungselemente 12 bilden so in der Draufsicht im wesentlichen ein gleichmäßiges Achteck. Es hat sich gezeigt, daß durch diese bevorzugte Ausgestaltung eine gute Stabilisierung der Wandung 6 erzielt werden kann. Selbstverständlich können je nach Größe und Form des Gasraumes 4 jedoch auch mehr oder weniger Stabilisierungselemente 12 vorgesehen sein.

Die Umlenkrollen 16 sind dabei jeweils als Walzen 16 ausgebildet, um die jeweils das Stabilisierungselement 12 geführt wird. Den Walzen 16 ist jeweils eine weitere Umlenkrolle 24 zugeordnet, die im Ausführungsbeispiel ebenfalls als Walze 24 ausgebildet ist. Es ist jedoch auch möglich, eine der Umlenkrollen 16, 24 als Walze und die andere nicht als Walze auszubilden. Die weiteren Walzen 24 sind jeweils zwischen zwei Stützen 28 befestigt.

Der Gasspeicher 2 weist auch hier eine im wesentlichen zylindrische Wandung 6 auf. Der Gasspeicher 2 besteht aus einem in der Draufsicht achteckigen Deckengestell 36 und einer Deckenmembran 38, die mit der Wandung 6 gasdicht verbunden ist. Zur besseren Übersicht ist die Deckenmembran 38 in Fig. 3 durchsichtig gestaltet, um die darunterliegende Lage der Wandung 6 andeuten zu können.

An der Decke 8 sind in Verlängerung des Deckengestells 36 jeweils Streben 30 angebracht, an denen endseitig die Führungsmittel 32 in der in Fig. 1 und 2 dargestellten Art angeordnet sind. Auch hier werden die Führungsmittel 32 bevorzugt in Form von zwei übereinanderlaufenden Rollen gebildet. Die Führungsmittel 32 sind an Führungsschienen 34 verfahrbar, die jeweils an den Stützen 28 angeordnet sind.

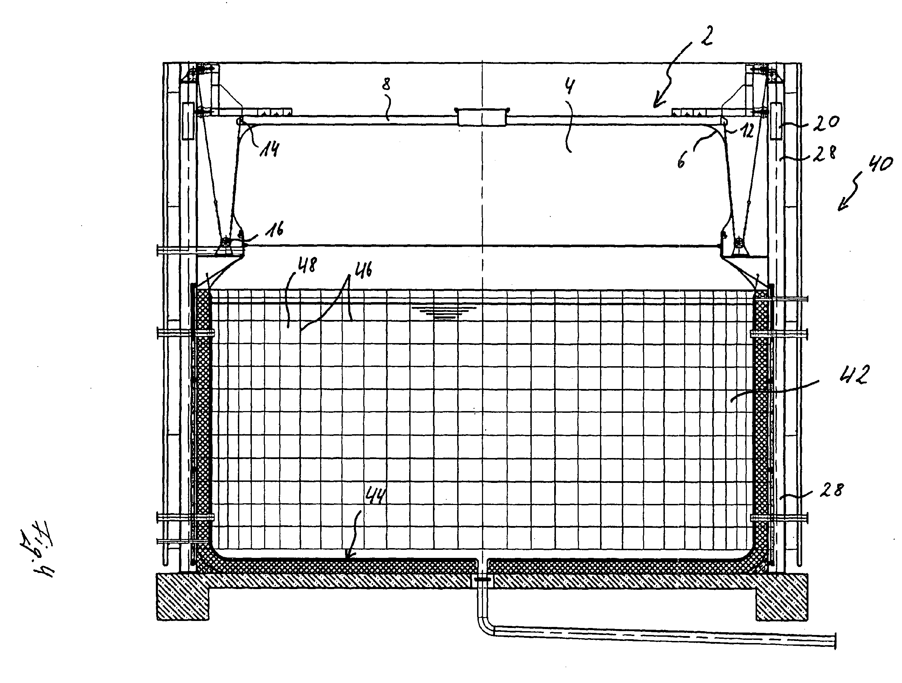

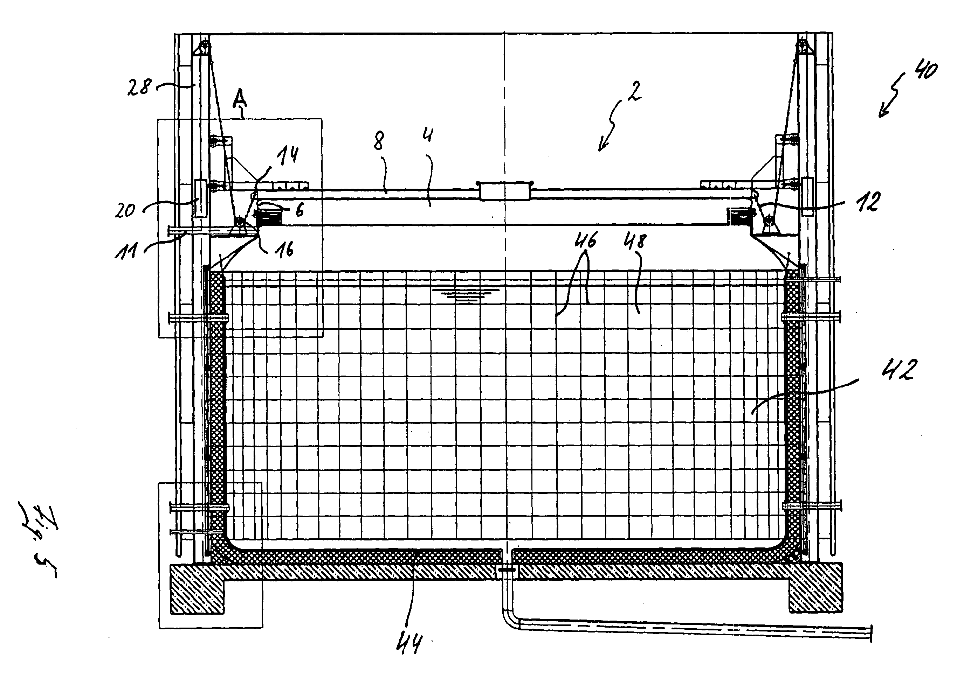

Fig. 4 und 5 zeigen eine erfindungsgemäße Biogasanlage 40 mit einem Gärbehälter 42 oberhalb dessen ein Gasspeicher 2 der zuvor beschriebenen Art angeordnet ist. Der Gasspeicher 2 ist mit dem Gärbehälter 42 gasdicht verbunden und in Fig. 4 voller als in Fig. 5. Der Kopfraum des Gärbehälters 42 bzw. der Biogasanlage 40 wird vom Gasspeicher 2 gebildet. Der Gasraum 4 des Gasspeichers 2 wird nach unten hin entweder vom Boden 44 oder aber von einer im Gärbehälter befindlichen Flüssigkeit, zum Beispiel Gülle, begrenzt. In dem Gärbehälter 42 befindet sich eine Fermentationslösung, wie z.B. Gülle oder Jauche, aus der über Fermentationsprozesse Biogas, insbesondere Methan, gebildet wird. Das Biogas wird dann im Gasspeicher 2 bevorzugt unter Druck gespeichert.

Zweckmäßigerweise werden die Stützen 28 des Gasspeichers 2 gleichzeitig als Stützen des Gärbehälters 42 verwendet. Zwischen die Stützen 28 ist dabei eine Gitterkonstruktion 46 angeordnet, die innenseitig mit einer flüssigkeitsdichten Gärbehältermembran 48 ausgelegt ist. Durch eine derartige Anordnung kann die Biogasanlage 40 mit leichten Materialien und in einfacher Weise schnell aufgebaut werden.

Bedingt durch die gegenüber der Umgebung erhöhte Lage des Gasspeichers 2 kann die als weiteres Widerlager 14 wirkende Fixierung der Stabilisierungselemente 12 auch nicht an den beweglichen Teilen Decke 8 oder Wandung 6 erfolgen, sondern beispielsweise am Gärbehälter 42. Die weiteren Widerlager 16 können dann als Umlenkrollen ausgebildet an der Decke fixiert werden. Hierdurch kann auf die weiteren Umlenkrollen 24 verzichtet werden.

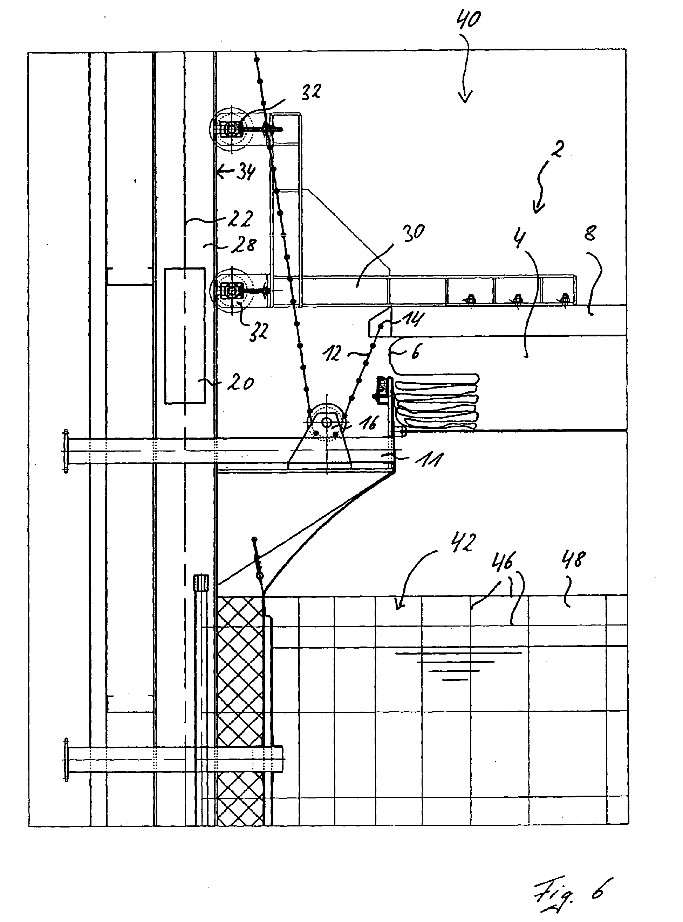

Fig. 6 zeigt die Vergrößerung des Ausschnitts A aus Fig. 5. Der Gasspeicher 2 weist prinzipiell die gleichen Elemente auf, die auch in Fig. 1 und 2 dargestellt sind. Der Gasspeicher 2 ist gasdicht mit dem Gärbehälter 42 verbunden.

## Patentansprüche

1. Gasspeicher (2), mit einem größenvariablen Gasraum (4), der zumindest bereichsweise von einer flexiblen Wandung (6) und einer Decke (8) als beweglichen Teilen begrenzt ist, mit wenigstens einem die Ausdehnung der Wandung (6) zumindest bereichsweise begrenzenden Stabilisierungselement (12), das flexibel ist,und mit zumindest einer Spanneinrichtung mit wenigstens einem Paar zusammenwirkender Widerlager (14, 16) zwischen denen zumindest ein Teil des Stabilisierungselements (12) durch Zugspannung gestrafft ist, wobei lediglich eines der jeweils zusammenwirkenden Widerlager (14, 16) fest mit einem der beweglichen Teile (6, 8) verbunden ist, **dadurch gekennzeichnet, dass** das erste Widerlager des zusammenwirkenden Widerlagerpaares (14, 16) von einer Umlenkrolle (16) gebildet wird und die Spanneinrichtung je Umlenkrolle (16) wenigstens eine weitere Umlenkrolle (24) aufweist, um die das Stabilisierungselement (12) direkt oder indirekt über ein an dem Stabilisierungselement (12) angreifendes Zugelement (22) geführt ist, wobei die weitere Umlenkrolle (24) weiter vom Boden (10) entfernt angeordnet ist als die erste Umlenkrolle (16).

2. Gasspeicher (2) nach Anspruch 1, **dadurch gekennzeichnet, daß** das Stabilisierungselement (12) ein Netz, ein Seil, ein Gewebe, eine Folie oder dergleichen oder eine Kombination zweier oder mehrerer dieser Elemente ist.

3. Gasspeicher (2) nach einem der vorgehenden Ansprüche, **dadurch gekennzeichnet, daß** die Spanneinrichtung das Stabilisierungselement (12) mit einer Zugspannung im wesentlichen parallel zur Wandung (6) des Gasspeichers (2) beaufschlagt.

4. Gasspeicher (2) nach einem der vorgehenden Ansprüche, **dadurch gekennzeichnet, daß** die Spanneinrichtung zumindest ein Zuggewicht (20) aufweist, das zur Aufbringung der Zugkraft direkt oder indirekt über ein Zugelement (22) an dem Stabilisierungselement (12) angreift, wobei das Stabilisierungselement (12) oder das Zugelement (22) um die Umlenkrolle (16) geführt ist.

5. Gasspeicher (2) nach einem der vorgehenden Ansprüche, **dadurch gekennzeichnet, daß** das weitere Widerlager (14) des zusammenwirkenden Widerlagerpaares (14, 16) an der Decke (8) fixiert und die Umlenkrolle (16) unterhalb des weiteren Widerlagers (14) angeordnet ist.

6. Gasspeicher (2) nach einem der vorgehenden Ansprüche, **dadurch gekennzeichnet, daß** die weitere Umlenkrolle (24) an einer Stütze (28) befestigt ist.

7. Gasspeicher (2) nach einem einem der vorgehenden Ansprüche, **dadurch gekennzeichnet, daß** die erste Umlenkrolle (16) eine Walze ist, um die das Stabilisierungselement (12) geführt ist.

8. Gasspeicher (2) nach einem der Ansprüche 1 bis 6, **dadurch gekennzeichnet, daß** der Gasspeicher (2) acht Stabilisierungselemente (12) und acht erste Umlenkrollen (16) aufweist.

9. Gasspeicher (2) nach Anspruch 8, **dadurch gekennzeichnet, daß** der Gasspeicher (2) acht Stützen (28) und acht weitere Umlenkrollen (24) aufweist.

10. Gasspeicher (2) nach einem der vorgehenden Ansprüche, **dadurch gekennzeichnet, daß** die Decke (8) wenigstens eine Halterung für zusätzliche Gewichte aufweist.

11. Gasspeicher (2) nach einem der vorgehenden Ansprüche, **dadurch gekennzeichnet, daß** an der Decke (8) wenigstens zwei Streben (30) mit endseitigen Führungsmitteln (32) angeordnet sind, die entlang jeweils zugeordneter und komplementär zu den Führungsmitteln (32) ausgebildeter Führungsschienen (34) vertikal verfahrbar sind.

12. Gasspeicher (2) nach einem der vorhergehenden Ansprüche, **gekennzeichnet durch** wenigstens eine mit der Wandung (6) verbundene oder als Teil der Wandung (6) ausgebildete Falthilfe, zur gleichmäßigen Faltung der Wandung (6) bei einem sich verkleinernden Gasraum (4).

13. Biogasanlage (40) zur Erzeugung von Biogas, insbesondere Methan, mit einem Gärbehälter (42) und einem Gasspeicher (2) nach einem der vorhergehenden Ansprache, der mit dem Gärbehälter (42) gasdicht verbunden ist.

14. Biogasanlage (40) nach Anspruch 13, **dadurch gekennzeichnet, daß** der Gasspeicher (2) oberhalb des Gärbehälters (42) angeordnet ist.

## Claims

1. Gas reservoir (2), having a gas chamber (4), the size of which can be varied and at least a region or regions of which are defined by a flexible wall (6) and a roof (8) acting as movable parts, having at least one stabilising element (12) which is flexible and which limits the expansion of the wall (6) at least in a region or regions, and having at least one tensioning device having at least one pair of co-operating abutments (14, 16) between which at least a part of the stabilising element (12) is held taut by tensile stress, only one of the abutments (14, 16) which co-operate in each case being fixed to one of the movable parts (6, 8), **characterised in that** the first abutment of the co-operating pair of abutments (14, 16) is formed by a return pulley (16) and the tensioning device has, for each return pulley (16), at least one further return pulley (24) around which the stabilising element (12) is passed, directly or indirectly, by means of a tension-applying member (22) which engages with the stabilising member (12), the further return pulley (24) being arranged further away from the ground (10) than the first return pulley (16).

2. Gas reservoir (2) according to claim 1, **characterised in that** the stabilising element (12) is a net, a cable, a woven fabric, a film or foil or the like or a combination of two or more of these items.

3. Gas reservoir (2) according to one of the preceding claims, **characterised in that** the tensioning device applies a tensile stress to the stabilising element (12) substantially parallel to the wall (6) of the gas reservoir (2).

4. Gas reservoir (2) according to one of the preceding claims, **characterised in that** the tensioning device has at least one tension-applying weight (20) which, to apply the tensioning force, engages directly or indirectly with the stabilising element (12) via a tension-applying member (22), the stabilising element (12) or the tension-applying member (22) passing round the return pulley (16).

5. Gas reservoir (2) according to one of the preceding claims, **characterised in that** the further abutment (14) of the pair of co-operating abutments (14, 16) is fixed to the roof (8) and the return pulley (1E) is arranged below the further abutment (14).

6. Gas reservoir (2) according to one of the preceding claims, **characterised in that** the further return pulley (24) is fastened to an upright (28).

7. Gas reservoir (2) according to one of the preceding claims, **characterised in that** the first return pulley (16) is a roller around which the stabilising element (12) is passed.

8. Gas reservoir (2) according to one of claims 1 to 6, **characterised in that** the gas reservoir (2) has eight stabilising elements (12) and eight first return pulleys (16).

9. Gas reservoir (2) according to claim 8, **characterised in that** the gas reservoir (2) has eight uprights (28) and eight further return pulleys (24).

10. Gas reservoir (2) according to one of the preceding claims, **characterised in that** the roof (8) has at least one mounting for additional weights.

11. Gas reservoir (2) according to one of the preceding claims, **characterised in that** there are arranged on the roof (8) at least two struts (30) having at their ends guiding means (32) which can be displaced vertically along respective associated guide rails (34) which are of a form complementary to the guiding means (32).

12. Gas reservoir (2) according to one of the preceding claims, **characterised by** at least one folding aid which is connected to the wall (6) or is formed as part of the wall (6), to allow the wall (6) to be folded evenly as the gas chamber (4) becomes smaller.

13. Biogas plant (40) for generating biogas, and in particular methane, having a digester tank (42) and a gas reservoir (2) according to one of the preceding claims which has a gastight connection to the digester tank (42).

14. Biogas plant (40) according to claim 13, **characterised in that** the gas reservoir (2) is arranged above the digester tank (42).

## Revendications

1. Réservoir de gaz (2), avec une chambre de gaz (4) de taille variable qui est délimitée au moins par secteur par une paroi souple (6) et un plafond (8) sous forme de pièces mobiles, avec au moins un élément de stabilisation (12) souple délimitant au moins par secteur l'extension de la paroi (6) et avec au moins un dispositif de tension avec au moins une paire de contre-appuis coopérants (14, 16) entre lesquels au moins une partie de l'élément de stabilisation (12) est tendue par un effort de traction, dans lequel seul l'un des contre-appuis respectivement coopérants (14, 16) est relié solidement à l'une des pièces mobiles (6, 8), **caractérisé en ce que** le premier contre-appui de la paire de contre-appuis coopérants (14, 16) est formé par une poulie de renvoi (16) et le dispositif de tension par poulie de renvoi (16) présente au moins une autre poulie de renvoi (24) autour de laquelle l'élément de stabilisation (12) est guidé directement ou indirectement par le biais d'un élément de traction (22) venant en prise sur l'élément de stabilisation (12), dans lequel l'autre poulie de renvoi (24) est plus éloignée du sol (10) que la première poulie de renvoi (16).

2. Réservoir de gaz (2) selon la revendication 1, **caractérisé en ce que** l'élément de stabilisation (12) est un filet, un câble, un tissu, un film ou similaire ou une combinaison de deux ou plusieurs de ces éléments.

3. Réservoir de gaz (2) selon l'une quelconque des revendications précédentes, **caractérisé en ce que** le dispositif de tension sollicite l'élément de stabilisation (12) par un effort de traction essentiellement parallèle à la paroi (6) du réservoir de gaz (2).

4. Réservoir de gaz (2) selon l'une quelconque des revendications précédentes, **caractérisé en ce que** le dispositif de tension présente au moins un poids de traction (20) qui vient en prise sur l'élément de stabilisation (12) directement ou indirectement par le biais d'un élément de traction (22) pour l'application de l'effort de traction, dans lequel l'élément de stabilisation (12) ou l'élément de traction (22) est guidé autour de la poulie de renvoi (16).

5. Réservoir de gaz (2) selon l'une quelconque des revendications précédentes, **caractérisé en ce que** l'autre contre-appui (14) de la paire de contre-appuis coopérants (14, 16) est fixé au plafond (8) et la poulie de renvoi (16) est disposée sous l'autre contre-appui (14).

6. Réservoir de gaz (2) selon l'une quelconque des revendications précédentes, **caractérisé en ce que** l'autre poulie de renvoi (24) est fixée à un support (28).

7. Réservoir de gaz (2) selon l'une quelconque des revendications précédentes, **caractérisé en ce que** la première poulie de renvoi (16) est un cylindre autour duquel l'élément de stabilisation (12) est guidé.

8. Réservoir de gaz (2) selon l'une quelconque des revendications 1 à 6, **caractérisé en ce que** le réservoir de gaz (2) présente huit éléments de stabilisation (12) et huit premières poulies de renvoi (16).

9. Réservoir de gaz (2) selon la revendication 8, **caractérisé en ce que** le réservoir de gaz (2) présente huit supports (28) et huit autres poulies de renvoi (24).

10. Réservoir de gaz (2) selon l'une quelconque des revendications précédentes, **caractérisé en ce que** le plafond (8) présente au moins une fixation pour des poids supplémentaires.

11. Réservoir de gaz (2) selon l'une quelconque des revendications précédentes, **caractérisé en ce qu'**au moins deux entretoises (30) avec des moyens de guidage (32) côté extrémité, qui sont déplaçables verticalement le long de rails de guidage (34) respectivement affectés et réalisés de manière complémentaire aux moyens de guidage (32), sont disposés au niveau du plafond (8).

12. Réservoir de gaz (2) selon l'une quelconque des revendications précédentes, **caractérisé par** au moins une aide au plissage reliée à la paroi (6) ou réalisée comme une partie de la paroi (6) pour un plissage régulier de la paroi (6) lors d'une réduction de la taille de la chambre de gaz (4).

13. Installation de biogaz (40) permettant de produire du biogaz, en particulier du méthane, avec un fermenteur (42) et un réservoir de gaz (2) selon l'une quelconque des revendications précédentes, qui est relié au fermenteur (42) de manière étanche aux gaz.

14. Installation de biogaz (40) selon la revendication 13, **caractérisée en ce que** le réservoir de gaz (2) est disposé au-dessus du fermenteur (42).
